Europäisches Patentamt

European Patent Office

Office européen des brevets

(11) Publication number: **0 376 939 B1**

(12) **EUROPEAN PATENT SPECIFICATION**

(45) Date of publication of patent specification: **08.07.92**  (51) Int. Cl.⁵: **A61B 17/60,** A61F 5/04, F16C 11/10

(21) Application number: **86902949.6**

(22) Date of filing: **29.04.86**

(86) International application number:
**PCT/SE86/00198**

(87) International publication number:
**WO 86/06270 (06.11.86 86/24)**

(54) **External fixation device.**

(30) Priority: **02.05.85 SE 8502134**

(43) Date of publication of application:
**11.07.90 Bulletin 90/28**

(45) Publication of the grant of the patent:
**08.07.92 Bulletin 92/28**

(84) Designated Contracting States:
**AT BE CH DE FR GB IT LI LU NL SE**

(56) References cited:
WO-A-83/02554    DE-A- 2 213 684
DE-B- 2 847 006    FR-A- 1 203 285
SE-A- 108 346    US-A- 1 468 659
US-A- 4 135 505

(73) Proprietor: **HAMMER, Richard
Stentorpsvägen 18
S-582 69 Linköping(SE)**

(72) Inventor: **HAMMER, Richard
Stentorpsvägen 18
S-582 69 Linköping(SE)**

(74) Representative: **Westerlund, Christer et al
L.A. Groth & Co Patentbyra AB, P.O. Box
6107
S-102 32 Stockholm(SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 376 939 B1

## Description

### Technical Field

The present invention relates to an arrangement in instrumentaria for fixating and stabilizing fractured skeletal parts and being of the kind set forth in the pre-characterizing clause of Claim 1.

### Background Prior Art

Instrumentaria of the aforesaid kind and similar kinds are known in the art, see for example in this regard pages 4-11 in the publication "Complications of External Skeletal Fixation" published by Charles C. Thomas, Springfield, Illinois, USA. Of the instrumentaria described in this publication, the instrumentarium most used is that proposed by Hoffmann (vide Figure 6 and 9), although this instrumentarium is encumbered with a number of drawbacks, of which the most serious resides in the fact that considerable manipulating forces are required firstly to lock a first part of a holding device, said part being lockable in turn to a connecting rod, to a second rotatable part of said device, and to lock the pins to said second part. A further drawback resides in undesirable sliding and/or rotation between mutually locked parts of the instrumentarium when large forces are applied thereto.

The US Patent Specification 4 135 505 describes and illustrates an instrumentarium in which the connection between a connecting rod or bar and a pin-holding device comprises a ball-and-socket coupling, the ball of which is introduced between and lies against ball seatings in the form of bevelled surfaces, the ball seatings being urged into engagement with the ball by means of screws. In a more recent instrumentarium, designated ORTHOFIX (Registered Trade Mark) the connection between a connecting between a connecting rod and a pinholding device comprises a ball-and-socket coupling, in which the ball is introduced between and lies against the ball seatings which present abutment surfaces in the form of part-spherical surfaces,wherewith the one seating can be urged against the ball by means of a rotatable cam shaft.

In a further instrumentarium, proposed in recent times and manufactured and retailed by EX-FI-RE systems, Kristiansand, Norway, the connection between a part of a holding device,which is capable of being locked to a connecting rod, and a pin-locking part of said arrangement comprises a ball-and-socket coupling. A knurled or servated ball is introduced between a first seating, in the form of a part-spherical surface, with surface abutment between the ball and seating, and a second seating in the form of a hollow nut, which is screwed onto the first seating in a manner to urge the seatings towards one another and therewith lock the ball therebetween.

The three last mentioned instrumentaria are encumbered essentially with the same disadvantages as those described with reference to the instrumentarium proposed by Hoffmann.

### Summary of the Invention

It is the object of the present invention to eliminate at least partially the drawbacks encountered with known instrumentaria of the aforedescribed kind, and to provide an improvement in instrumentaria which enables the two parts of the holding device to be locked together and the one part of the holding device to be locked to said pins with the aid of relatively small actuating forces, and which improvement will ensure that the said parts remain firmly locked when the instrumentarium is in use, and that the various components of the holding device are capable of being adjusted to a selected one of a large number of possible positions in relation to one another and in relation to the skeletal parts to be healed, and that these procedures can be carried out in a simple and ready manner.

This object is fulfilled with the arrangement according to the invention which has the characterizing features set forth in the characterizing clauses of the following Claims.

### Brief Description of the Drawings

Figure 1 illustrates in perspective the leg of a human being and an instrumentarium for the fixation and stabilization of fractured skeletal parts of the bone, this instrumentarium incorporating the arrangement according to the invention; and

Figures 2 and 3 are exploded views of parts of the arrangement illustrated in Figure 1.

### Description of a Preferred Embodiment

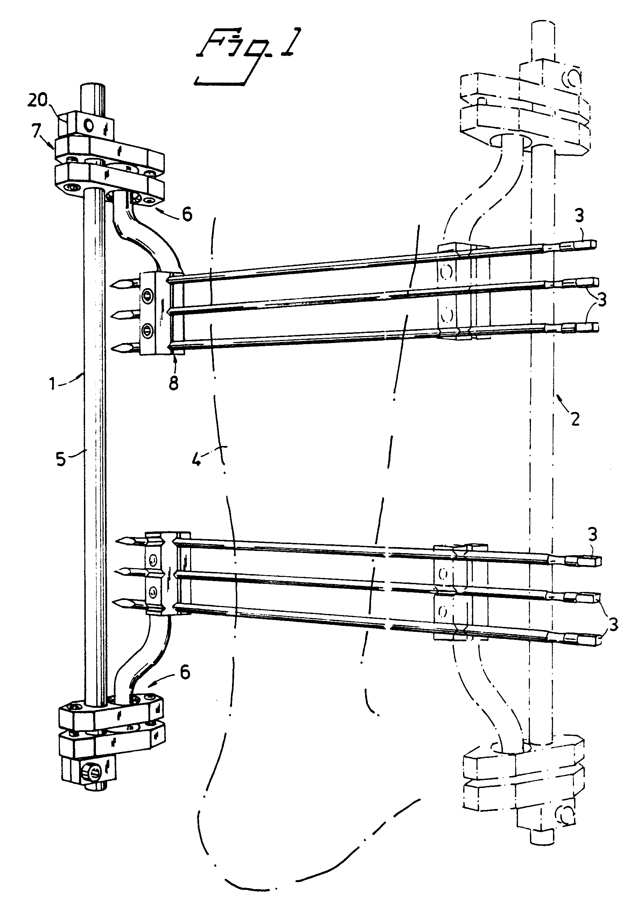

Figure 1 illustrates an instrumentarium which comprises two mutually identical assemblies 1 and 2 having attached thereto and extending therebetween identical pins 3. The pins 3 are of substantially circular cross-section and are inserted into prebored holes in fractured parts of the bone of a leg 4, illustrated schematically in chain lines, so as to fixate and stabilize the fractured bone until healed. It will be understood, of course, that the instrumentarium can be used in conjunction with skeletal parts other than the bones of a leg, and that at times it may be sufficient to use solely one of the illustrated assemblies 1 and 2, together with the pins 3, as a complete instrumentarium.

The construction of the assembly 1, the manner in which it is mounted and its method of use will now be described in greater detail.

The Assembly 1 comprises three major components, namely a connecting device in the form of a rectalinear steel tube and two mutually identical holding devices 6, each of which comprises a first part 7, which is constructed for axial movement along the tube 5 and can be locked to the tube in selected positions therealong, and a second part 8, which is rotatably connected to the first part 7 and capable of being locked thereto and which is provided with pin locking means, described in more details hereinafter.

The first part 7 of the assembly 1 comprises a steel plate 9 having provided therein four mutually parallel holes 10, 11, 12 and 13, of which only the hole 13 is provided with a screwthread, and a steel plate 14 having provided therein four mutually parallel holes 15, 16, 17 and 18, of which the hole 15 is provided with a screwthread, and with a fifth hole 19 the axis of which extends vertically to the axes of the lastmentioned holes and which intersects a slot 20 (vide Figure 1) extending between the hole 16 and the outer surface of the plate. Only that part of the hole 19 which is located on one side of the slot 20 is screwthreaded.

The hole 12 in the plate 9 is defined by a cylindrical inner wall 12a having a sharp annular edge 12b. The hole 17 in the plate 14 is defined by a cylindrical inner wall 17a having a sharp annular edge 17b.

The sharp edges 12b and 17b have a locking function, which will be described in more detail hereinafter, and in order to retain an effective edge locking action, even after having used the instrumentarium for a long period of time, the edges must be hard and wear resistant. This is achieved by hardening the plates 9 and 12, at least in the regions of the edges 12b, 17b of respective holes 12, 17, or alternatively by forming said edges on separate annular inserts and subsequently press-fitting the inserts into irrespective holes. The edges 12b, 17b consist of stainless, acid-proof steel and have a hardness of 220-250 Brinell.

The other part 8 of the assembly 1 comprises a steel shaft 21 which has the form of a crank-like lever and which has located on one end thereof a stainless steel ball 22 having a smooth outer surface which is hardened to a Brinell hardness of 220-250, and the other end of which shaft has located thereon a substantially parallelepipedic body 23 which is provided with three V-shaped recesses or grooves 24 on one side thereof and with three V-shaped recesses or grooves 25 on the opposite side thereof, and further comprises a parallelepipedic body 26 which has three V-shaped recesses of grooves 27 on one side thereof. The bodies 23 and 26 are provided with respective holes 28 and 29, of which only the holes 28 are provided with screwthreads.

The second part 8 of the assembly 1 is intended to be fitted to the first assembly part 7, by passing the second part 8, without the body 26, through the hole 12 in the plate 9-with the body 23 leading until the ball 22 lies against the annular edge 12b, the diameter of the ball being larger than that of said annular edge. The plate 14 is then securely fitted to the plate 9 with the aid of screws (not shown), which are passed from two directions through the holes 10, 13, 15, 18, wherewith the ball 22 abuts the annular edge 17b, the diameter of the ball being larger than that of said edge. When tightening the screws, the screw inserted through the mutually opposing holes 13, 18 is tightened to an extent which is assumed to correspond to the extent to which all said screws are to be finally tightened, whereas the remaining screws are tightened to an extent which is just sufficient to enable the ball 22, which thus lies against the mutually spaced plates 9 and 14 solely in line abutment with resprective edges 12b and 17b, is able to rotate and pivot upon manual activation of the shaft 21.

The thus assembled parts 7 and 8 are then fitted to the tube 5, which passes through the holes 11 and 16 in respective parts, and a screw (not shown) is screwed into the hole 19 and tightened sufficiently to urge together the two parts of the plate 14 located on opposite sides of the slot 20, so as to loosely clamp the assembly to the tube.

A further assembly, comprising first and second parts 7 and 8 and assembled in a manner similar to the first-mentioned assembly, is then fitted to the tube 5 and the positions of the two assemblies are adjusted in relation to the positions of the pins 3 previously inserted in the skeletal parts to be healed, such that the pins lie in the aforesaid recesses or grooves 24 or 25.

This adjustment to the positions of the two assemblies is effected by displacing the parts 7 and 8 axially along the tube 5 and by rotating and/or pivoting the shaft 21, which due to its cranklike configuration enables a suitable position of adjustment to be achieved from a plurality of possible adjustment positions, such that the instrumentarium is as stable as possible and the various screws can be readily reached for subsequent manipulation. The body 26 is then fitted to the body 23, with the recess 27 facing either the recess 24 or the recess 25. Which of the recesses 24 or 25 is chosen depends on the accessability of said recess and/or the accessability of the screws (not shown), which are inserted through the holes 28 and 29 and which urge the bodies 23, 26 towards one another so that the selected recess 24 or 25 and the recess 27 are brought into line abutment with the pins 3, thereby requiring but a relatively small tightening force on the screws.

Subsequent to making minor adjustments to the settings of the assemblies 6, the screw passing through the holes 10, 15 and connecting the plates 9 and 14 is tightened, the holes 10, 15 being located at a further distance from the ball-and-socket coupling 12b, 17b, 22 than the holes 13, 18, whereby the annular edges 12b and 17b are pressed into line abutment with the spherical surface of the ball 22, which means that the force required to tighten the assembly and the locking force achieved are respectively smaller and greater than the comparable forces required and achieved in the case of a surface abutment.

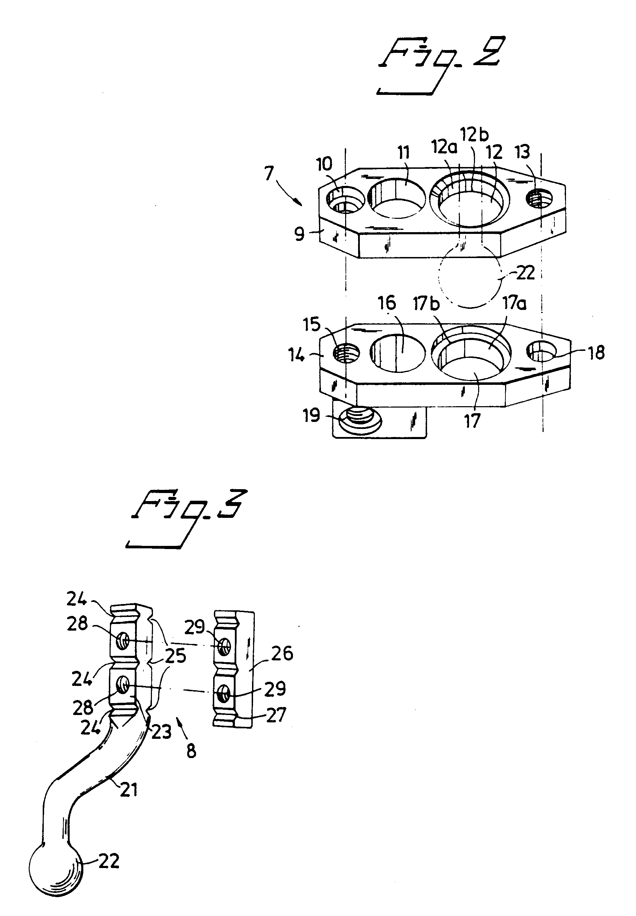

Solely the material from which the ball 22 and the plates 9 and 14, and the sharp annular edges 12b, 17b are made has a significance in achieving an optimal locking effect. The positioning of the two annular abutment lines between the said edges and the ball is also important however. It has been found that when the ball and the plates are made from the aforedescribed material, the abutment lines should be spaced at a short distance apart. In this regard, a radius drawn from the centre of the ball to the tangent of each abutment line to the spherical surface of the ball should form, together with a line passing through the centre of the ball and located in the vertical plane in Figure 2, a maximum and also an optimum angle of about 67.3°. When this angle is exceeded, the surface of the ball will be plastically deformed by the shear forces generated, therewith impairing the locking effect. In conjunction with the aforesaid critical, optimum angle the ratio between the diameter of each annular edge 12b, 17b and the diameter of the ball 22 is 0.90-0.91.

In tests carried out with identical materials in the ball 22 and the spherical edges 12b, 17b having a Brinell hardness of 220-250, there was achieved a maximum locking torque force, of 35Nm at optimum angles. This torque force was obtained when applying a tightening torque to the screw passed through the holes 10, 15, of 4 Nm and was not observed to increase with increased torque. With other conditions equal, the locking torque can be increased to above 50 Nm when the surface hardness of the edges and ball is selected so that the ratio is at least 2:1.

The relatively large lever arm effect between the ball-and-sock coupling and the holes 10, 15 enables a smaller torque to be applied than that required when the aforesaid distances are equal or when the screw inserted in the holes 13, 18 is tightened subsequent to tightening the screw inserted in the holes 10, 15.

Finally, the screws inserted through the holes 19 are fully tightened, wherewith the instrumentarium is able to fulfill its intended function.

## Claims

1. An arrangement in instrumentaria for external fixation and stabilization of fractured skeletal parts, comprising at least one connecting element (5) which extends along said skeletal parts and on which there is mounted at least one pin-holding device (6) for firmly holding a pin (3) inserted in each skeletal part, wherewith a first assembly part (7) of at least the one holding device is displaceable along the connecting element and capable of being locked thereto in selected positions therealong, and wherewith a second assembly part (8) of the pin-holding device is rotatably connected with and lockable to the first assembly part by means of a ball-and-socket coupling and is provided with means (23-29) for locking the pins, **characterized** in that the ball-and-socket coupling has line abutment between the ball (22) and a sharp annular edge (12b, 17b) on each of the ball seatings against which the ball can be urged.

2. An arrangement according to Claim 1, **characterized** in that the linear abutment extends along two substantially parallel circular lines on the surface of the ball (22), irrespective of the position to which the ball is rotated and swung in relation to the ball seats (12b, 17b).

3. An arrangement according to Claim 1 or 2, **characterized** in that the ratio between the diameter of each edge (12b, 17b) and the diameter of the ball (22) is approximately 0.9.

4. An arrangement according to any of the preceding Claims, **characterized** in that the ball seatings (12b, 17b) can be urged into engagement with the ball (22) by means of devices (at 10, 15 and 13, 18 respectively) connecting said seatings, of which elements one is located at a greater distance from the ball than the other.

5. An arrangement according to any of the preceding Claims, **characterized** in that the ball (22) is mounted on the second assembly part (8), which has the form of a preferably crank-like arm (21) which projects from the first assembly part (7) and the end of which remote from the ball is provided with said means (23-29) for locking the pins (3).

6. An arrangement according to any of the preceding Claims, **characterized** in that the means (23-29) for locking the pins (3) incorporates two locking devices (23, 26) which can be urged towards one another and which are provided with mutually facing V-shaped grooves (24, 25, 27) into which the pins are inserted and pressed thereagainst in line abutment therewith.

7. An arrangement according to Claims 5 and 6, **characterized** in that the one means (23) constitutes a part of the aforesaid second assembly part (8) and is provided with at least one V-chaped groove (24 and 25 respectively) on two mutually opposing sides thereof.

## Revendications

1. Appareil de fixation externe et de stabilisation de parties du squelette fracturées, comprenant au moins un élément de liaison (5) qui s'étend le long desdites parties du squelette et sur lequel est monté au moins un dispositif porte-broche (6) pour maintenir fermement une broche (3) insérée dans chaque partie du squelette, dans lequel un premier organe d'assemblage (7) d' au moins un dispositif porte-broche peut se déplacer le long de l'élément de liaison et peut être fixé sur ce dernier dans des positions sélectionnées le long de celui-ci, et dans lequel un deuxième organe d'assemblage (8) du dispositif porte-broche est relié au premier organe d'assemblage, de manière à pouvoir pivoter, et à pouvoir être immobilisé par rapport à lui, au moyen d'un pivot à rotule, et est pourvu de moyens (23-29) pour fixer les broches, caractérisé en ce que la rotule (22) du pivot à rotule vient buter de manière linéaire contre un bord annulaire en angle vif (12b, 17b) de chacun des sièges de rotule, contre lesquels la rotule peut être maintenue serrée.

2. Appareil selon la revendication 1, caractérisé en ce que la butée linéaire s'étend le long de deux lignes circulaires sensiblement parallèles situées à la surface de la rotule (22), quel que soit le sens de rotation et d'inclinaison de la rotule par rapport aux sièges de rotule (12b, 17b).

3. Appareil selon la revendication 1 ou 2, caractérisé en ce que le rapport entre le diamètre de chaque bord (12b, 17b) et le diamètre de la rotule (22) est d'environ 0,9.

4. Appareil selon l'une quelconque des revendications précédentes, caractérisé en ce que les sièges de rotule (12b, 17b) peuvent être serrés autour de la rotule (22) au moyen de dispositifs (en 10, 15, et 13, 18 respectivement) reliant lesdits sièges, dont un élément est situé à plus grande distance de la rotule que l'autre élément.

5. Appareil selon l'une quelconque des revendications précédentes, caractérisé en ce que la rotule (22) est montée sur le deuxième organe d'assemblage (8), qui a, de préférence, la forme d'un bras coudé (21) qui s'avance à partir du premier organe d'assemblage (7) et dont l'extrémité éloignée de la rotule est pourvue desdits moyens (23-29) pour fixer les broches (3).

6. Appareil selon l'une quelconque des revendications précédentes, caractérisé en ce que le moyen (23-29) pour fixer les broches (3) comprend deux dispositifs de fixation (23, 26) qui peuvent être poussés l'un vers l'autre et qui sont pourvus de rainures en forme de V placées en regard (24, 25, 27) dans lesquelles les broches sont insérées et pressées de telle sorte qu'elles viennent en butée, de manière linéaire, contre ces dernières.

7. Appareil selon les revendications 5 et 6, caractérisé en ce que le moyen (23) constitue un élément du deuxième organe d'assemblage (8) sus-mentionné et est pourvu d'au moins une rainure en forme de V (24, et 25 respectivement) située sur deux côtés opposés de celui-ci.

## Patentansprüche

1. Anordnung bei Instrumenten zur externen Fixierung und Stabilisierung von gebrochenen Skeletteilen, umfassend wenigstens ein Verbindungselement (5), das sich entlang der Skeletteile erstreckt und auf dem zumindest eine Stabhalteeinrichtung (6) zum Festhalten eines in jeden Skeletteil eingeführten Stabes (3) befestigt ist, wobei ein erster Montageteil (7) zumindest der einen Halteeinrichtung entlang des Verbindungselementes verschiebbar und in gewählten Positionen entlang desselben verriegelbar ist, und wobei ein zweiter Montageteil (8) der Stabhalteeinrichtung mit dem ersten Montageteil drehbar verbunden und auf diesem mittels einer Kugel/Pfannen-Kupplung verriegelbar sowie mit Organen (23-29) zur Verriegelung der Stäbe versehen ist, dadurch gekennzeichnet, daß die Kugel/Pfannen-Kupplung auf jedem der Kugelsitze, gegen welche die Kugel gedrückt werden kann, zwischen der Kugel (22) und einer scharfen ringförmigen Kante (12b, 17b) eine linienförmige Abstützung hat.

2. Anordnung nach Patentanspruch 1, dadurch gekennzeichnet, daß sich die lineare Abstützung entlang zweier im wesentlichen kreisförmiger Linien auf der Oberfläche der Kugel (22) erstreckt, unabhängig von der Position in welche die Kugel gedreht und in bezug auf die Kugelsitze (12b, 17b) geschwenkt worden ist.

3. Anordnung nach Patentanspruch 1 oder 2, dadurch gekennzeichnet, daß das Verhältnis zwischen dem Durchmesser jeder Kante (12b, 17b) und dem Durchmesser der Kugel (22) etwa 0,9 beträgt.

4. Anordnung nach einem der vorhergehenden Patentansprüche, dadurch gekennzeichnet, daß die Kugelsitze (12b, 17b) mittels sie verbindender Elemente (bei 10, 15 bzw. 13, 18) mit der Kugel (22) in Eingriff drückbar sind, wobei eines dieser Elemente in größerem Abstand von der Kugel angeordnet ist als das andere.

5. Anordnung nach einem der vorhergehenden Patentansprüche, dadurch gekennzeichnet, daß die Kugel (22) auf dem zweiten Montageteil (8) angeordnet ist, der die Form eines vorzugsweise kurbelähnlichen Armes (21) aufweist, welcher vom ersten Montageteil (7) vorragt und dessen von der Kugel entferntes Ende mit den Organen (23-29) zur Verriegelung der Stäbe (3) versehen ist.

6. Anordnung nach einem der vorhergehenden Patentansprüche, dadurch gekennzeichnet, daß die Organe (23-29) zur Verriegelung der Stäbe (3) zwei Sperrelemente (23, 26) umfassen, die gegeneinander drückbar und mit gegenüberliegenden V-förmigen Nuten (24, 25, 27) versehen sind, in welche die Stäbe eingesetzt sind und in linearer Abstützung mit diesen dagegen angepreßt werden.

7. Anordnung nach Patentanspruch 5 und 6, dadurch gekennzeichnet, daß das eine Organ (23) einen Teil des zweiten Montageteils (8) bildet und mit wenigstens einer V-förmigen Nut (24 bzw. 25) an zwei entgegengesetzten Seiten desselben versehen ist.

Fig.1

Fig. 2

Fig. 3